(19) Europäisches Patentamt
European Patent Office
Office européen des brevets

(11) Publication number: **0 293 166 A1**

(12) **EUROPEAN PATENT APPLICATION**

(21) Application number: **88304681.5**

(22) Date of filing: **24.05.88**

(51) Int. Cl.⁴: **C07C 69/76 , C07C 67/08 , C07C 67/26**

(30) Priority: **26.05.87 US 53992**

(43) Date of publication of application: **30.11.88 Bulletin 88/48**

(84) Designated Contracting States: **DE FR IT**

(71) Applicant: **E.I. DU PONT DE NEMOURS AND COMPANY**
**Legal Department 1007 Market Street**
**Wilmington Delaware 19898(US)**

(72) Inventor: **Kirchner, Jack Robert**
**2423 Shellpot Drive**
**Wilmington Delaware 19803(US)**

(74) Representative: **Woodcraft, David Charles et al**
**BROOKES & MARTIN High Holborn House**
**52/54 High Holborn**
**London, WC1V 6SE(GB)**

(54) **Manufacture of fluoromellitates.**

(57) Process which comprises reacting a mixture of pyromellitic dianhydride, at least one perfluoroalkylethyl alcohol and epichlorohydrin in an organic base-containing solvent which is substantially inert to pyromellitic dianhydride, and recovering a mixture of monomeric bis-(n-perfluoroalkylethyl)-bis-(3-chloro-2-hydroxypropyl)-pyromellitates and at least one oligomeric product which is believed to be represented by the formula:

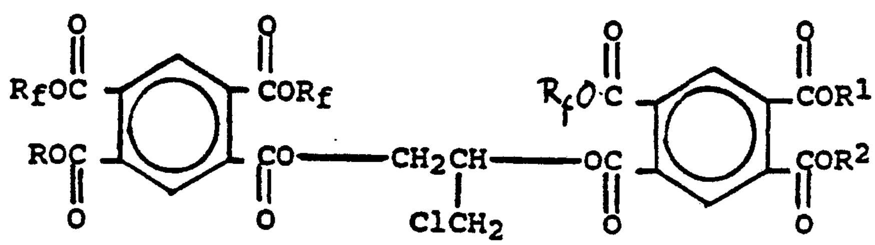

wherein
$R_f$ is $-CH_2CH_2(CF_2)_nCH_3$;
n is an integer from 3 to 17;
R is -

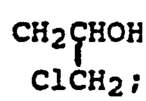

EP 0 293 166 A1

and

$R^1$ differs from $R^2$ and each is $R_f$ or R.

## MANUFACTURE OF FLUOROMELLITATES

The present invention relates to a process for the manufacture of bis-(n-perfluoroalkylethyl)-bis-(3-chloro-2-hydroxypropyl)-pyromellitates and oligomers thereof.

Mares et al., in U.S. Patent No. 4,209,610, disclose bis-(n-perfluoroalkylethyl)-bis-(3-chloro-2-hydroxypropyl)-pyromellitates and oligomers thereof, their manufacture, and use as water, oil and soil repellents. They first reacted pyromellitic dianhydride with a fluoroalcohol in dimethylformamide at a 2:1 mol ratio of alcohol:dianhydride, and when that reaction was essentially complete, epichlorohydrin was added to the reaction mixture at a 4:1 mol ratio of epichlorohydrin:dianhydride initially charged to the reactor. Oxenrider et al. carried out essentially the same reaction, substituting N-methylpyrrolidone for dimethylformamide, and using epichlorohydrin at a 6:1 mol ratio (U.S. Patent No. 4,321,403). Oxenrider discloses mixtures of fluorinated pyromellitate oligomers which were prepared by reacting pyromellitic dianhydride with a fluoroalcohol at a 1:2 mol ratio, and the resulting diester was reacted with an excess of epichlorohydrin to provide a tetraester which was reacted with additional pyromellitic dianhydride (U.S. Patent No. 4,414,277). Marshall et al., in U.S. patent No. 4,416,787, disclose yarn and spin finish compositions involving pyromellitates of the class described herein, and their application to synthetic yarn substrates, particularly those derived from polyesters and polyamides.

The process of the present invention comprises reacting a mixture of pyromellitic dianhydride, one or more perfluoroalkylethyl alcohols and epichlorohydrin in an organic base-containing solvent. In comparison to the prior art processes, the 1-step esterification process of this invention yields a more reproducible, higher quality product; the process is easier to control, requires fewer analyses and markedly shortens the process cycle time.

The process of this invention comprises reacting a mixture of pyromellitic dianhydride (PMDA), at least one perfluoroalkylethyl alcohol (PFA) and epichlorohydrin (ECH) in an organic base-containing solvent which is substantially inert to PMDA, and recovering a mixture of monomeric bis-(n-perfluoroalkylethyl)-bis(3-chloro-2-hydroxypropyl)-pyromellitates and at least one oligomeric product. In other words, bis-(n-perfluoroalkylethyl)-bis(3-chloro2-hydroxypropyl)-pyromellitates and oligomers thereof, used to impart water- and oil-repellency to polyethylene terephthalate or "nylon" fibers, were prepared by a single charge reaction of PMDA, PFA and ECH in an organic base-containing solvent. The reaction products were separated from excess ECH and/or water-soluble solvents used during their preparation and converted to a solvent-free, surfactant-stabilized aqueous dispersion by sequential washing with aqueous ammonia and water, with supplemental addition of an appropriate, poorly water-soluble solvent, if necessary, to maintain the product mixture in solution, emulsification of the washed product with water in the presence of a suitable surfactant, and subsequent removal of the organic solvent by reduced pressure distillation.

The reaction of the PFA, PMDA and ECH in the presence of a suitable catalyst and solvent is presumed to proceed via (1) the initial, and relatively fast, reaction of PFA and PMDA to form diester-diacid intermediates which (2) react with ECH at a relatively slower rate, in the presence of a suitable basic catalyst to yield a mixture of monomeric bis-(n-perfluoroalkylethyl)-bis-(3-chloro-2-hydroxypropyl)-pyromellitates (para and meta isomers as disclosed by Mares et al. in U.S. Patent 4,209,610) which (3) are subsequently converted at a relatively slow rate in the presence of a suitable basic catalyst to at least one oligomeric product. It not intended to limit the invention to any particular structure; however, it is believed that the oligomer is one which can be represented by the following formula:

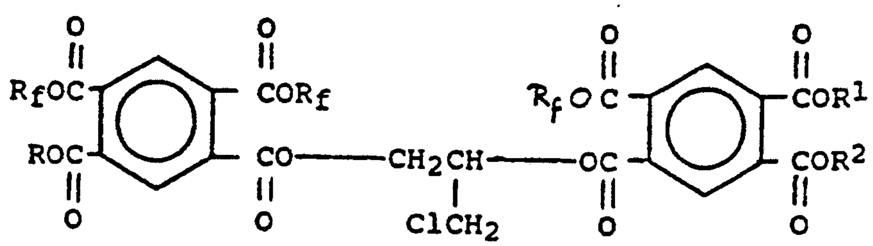

wherein:

$R_f$ is $-CH_2CH_2(CF_2)_nCF_3$;

n is an integer from 3 to 17;

R is $-CH_2CH(OH)CH_2Cl$; and

$R^1$ differs from $R^2$ and each is $R_f$ or R.

The multiple reaction sequence can be carried out over a range of time, temperature, solvent and catalyst type/concentration parameters and the monomer/oligomer distribution of the final product controlled to some degree by proper choice of reaction time/temperature and catalyst type/concentration parameters. Generally a temperature in the range between 70 and 130°C is suitable, with a temperature between 85 and 120°C being preferred. The reaction may be run over a period of three to 48 hours, usually between about three and 22 hours. Any solvent which is inert to PMDA (no active hydrogen) is suitable, e.g., N-methylpyrrolidone (NMP), methylisobutyl ketone (MIBK), methylethyl ketone, acetone, tetrahydrofuran, epichlorohydrin, as well as the ester solvents disclosed by Oxenrider et al. in U.S. Patent 4,321,403.

A mixture of MIBK and NMP is the preferred solvent. The preferred catalyst is triethylamine. However, a variety of tertiary amines can be used, e.g., pyridine, NMP, N-methylmorpholine, 2,6-lutidine.

Reasonably pure, dry reagents must be used for the product synthesis, since the presence of water and/or other PMDA reactive, non-fluorinated compounds in the reaction mixture adversely affect the final product composition and its ability to impart water and oil repellency to synthetic organic fibers. The reagents can be dried/purified individually or in appropriate admixture, prior to use by azeotrope distillation, fractional distillation, molecular sieves and/or other suitable means. The moisture content of the individual reagents and/or admixtures thereof can be conveniently determined by Karl-Fisher analysis and their purity levels by gas chromatography or other suitable analytical techniques.

The presence of a catalyst, typically a PMDA non-reactive organic base, such as triethyl amine (TEA), and/or optionally a weakly basic organic solvent, such as N-methyl-2-pyrrolidone (NMP), is required to (1) promote the ECH/carboxylic acid group esterification reaction and subsequent oligomerization of the resultant bis-(n-perfluoroalkylethyl)-bis-(3-chloro-2-hydroxypropyl)-pyromellitates and (2) if an organic ketone is used as a reaction solvent/diluent, to repress the acid-catalyzed reaction of the ketone solvent with ECH to form the corresponding ketal.

The specific basic reagent, or combination of basic reagents employed for the PFA/PMDA/ECH reaction step, and the relative concentrations thereof, is typically chosen to both insure the essentially complete esterification of all free carboxylic acid groups in the reaction mixture and obtain a desired monomer/oligomer distribution by the end of a preset time/temperature reaction period, and to repress ketal formation if an organic ketone were employed as a reaction solvent/diluent. The free carboxylic acid group esterification reaction can be monitored by standard titration procedures, the momomer/oligomer distribution of the product by high pressure liquid chromatography and ketal formation by gas chromatography.

The removal of unreacted ECH from the reaction mixture after the free carboxylic acid groups are essentially esterified (acid number ≤ 0.50) and a desired monomer/oligomer distribution is obtained, is conveniently accomplished by washing the reaction mixture with dilute ammonium hydroxide. Oligomerization of the PFA/PMDA/ECH reaction products will continue during the basic wash operation. The removal/destruction of the unreacted ECH can be monitored by gas chromatography.

The relative ability of a PFA/PMDA/ECH reaction product to impart oil and water repellency to synthetic organic fibers can be measured as per the general procedure of Examples XI and XII of U.S. 4,209,610. The water/oil repellency values reported hereinafter were obtained for product samples which were solvent-collecting (acetone/Freon® 113) at a fluorine level of about 1100 ppm to nylon 66 knit fabric and after the solvent-padded fabric was air dried, annealed 0.5 hour at 275°F, and subjected to nine home laundry wash cycles.

The following Examples further exemplify the invention. They illustrate the preparation of mixtures of bis-(n-perfluoroalkylethyl)-bis-(3-chloro-2-hydroxypropyl)-pyromellitates and oligomers thereof by the single charge reaction of PFA, PMDA and ECH in an organic base-containing solvent, and the conversion of said product to a surfactant-stabilized, essentially solvent-free aqueous dispersion. Oligomer and monomer contents were determined by high pressure liquid chromatography; see INTRODUCTION TO MODERN LIQUID CHROMATOGRAPHY, Chapter 12, by L.R. Snyder and J.J. Kirkland, published by John Wiley & Sons,inc.

## EXAMPLE 1

A 500 ml, 4-neck flask (glass) fitted with a paddle stirrer, thermometer, and Vigreaux column, Barrett trap, water condenser and drying tube was charged with 200.0 g (about 0.403 mol) of a wet mixture of 2-(n-perfluoroalkyl)ethanols having 4 to 18 carbon atoms in the perfluoroalkyl groups and containing isopropanol (IPA) as a minor impurity, 175.0 g of methylisobutyl ketone (MIBK), 6.67 g of NMP and 2.67 g of water. The resultant mixture was stirred and heated to and held at reflux with periodic withdrawal of the IPA/water and

MIBK/water azeotropes from the Barrett trap until the residual flask liquor contained 216 ppm of water by Karl-Fisher analysis. After the flask contents were cooled to about 45°C, the Vigreaux Column, Barrett trap and drying tube were removed from and an inlet tube for nitrogen gas added to the flask. The flask was then charged with 44.0 g (0.202 mol) of PMDA, 46.0 g (0.497 mol) of ECH and 1.72 g (0.017 mol) of TEA, a slow nitrogen purge was started to maintain a dry atmosphere, and the stirred mixture heated at a 5°C/10 minute rate to and then held at about 90°C for a total reaction period of 22 hours, after which time the acid number of the mixture was 0.32. The reaction product was added to a preheated 85°C solution of 5.87 g of reagent grade ammonium hydroxide in 482 g of water and stirred at about 85°C for two hours. After settling at about 85°C, the upper aqueous fraction was removed and replaced by 400 g of warm 85°C water containing 5.9 g of dissolved MIBK. The mixture was stirred an additional two hours at about 85°C, then settled at about 85°C for 16 hours. After the upper aqueous fraction was removed, the residual organic product solution was diluted with 50.1 g of MIBK. A 400 g portion of the diluted reaction product, 22.8 g of sodium lauryl sulfate and 212 g of water were emulsified, first using a Waring commercial blender and then a Gaulin Corporation laboratory homogenizer and sub-micron disperser. The homogenized mixture was vacuum-stripped of MIBK and some water at 23-50°C/93-96 mm Hg. for 55 minutes. The resultant, essentially solvent-free dispersion was water diluted to a density of 1.2015, as measured by a Metler Digital meter.

The monomer/oligomer ratio of the reaction product mixture as a function of the sequential preparative operations is tabulated below.

| Process Operation | Monomer/Oligomer Ratio |
|---|---|
| End 22 hour PFA/PMDA/ECH reaction | 40/60 |
| End 2 hour $NH_4OH$ wash | 36/64 |
| End 2 hour water wash | 35/65 |
| End 16 hour settle | 34/66 |
| End dispersed product water dilution | 34/66 |

## EXAMPLES 2 THROUGH 6

The general procedure and approximate reactant, catalyst and solvent weights of Example 1 were used to prepare single charge PFA/PMDA/ECH reaction products at varying reaction time/temperature parameters. The relative monomer/oligomer distribution and the MIBK/ECH derived ketal content of and the oil/water repellency imparted to nylon 66 fabric swatches by the corresponding dispersed products are tabulated below.

| Example | Reaction Temp. °C. | Reaction Time, Hrs. | Monomer/Oligomer Ratio |
|---|---|---|---|
| 2 | 90 | 22 | 35/65 |
| 3 | 105 | 18 | 39/61 |
| 4 | 120 | 4.5 | 40/60 |
| 5 | 120 | 20 | 34/66 |
| 6 | 120 | 22 | 29/71 |

| Example | Repellency Oil | Repellency Water | Ketal Percent |
|---|---|---|---|
| 2 | 5 | 5 | 0.01 |
| 3 | 5 | 6 | 0.05 |
| 4 | 6 | 7 | 0.11 |
| 5 | 6 | 6 | 0.06 |
| 6 | 6 | 7 | 0.06 |

## EXAMPLES 7 THROUGH 13

The general procedure and approximate reactant and NMP/MIBK weights of Example 1 were used to prepare a single charge PFA/PMDA/ECH reaction product at 120 °C. The reaction time and TEA weight charged was varied; the single charge reaction mixture was heated to 120 °C after admixture in about 25 minutes.

| Example | TEA Grams | Reaction Time, Hrs. | Monomer/Oligomer Ratio |
|---|---|---|---|
| 7 | 0.16 | 24 | 31/69 |
| 8 | 0.33 | 5 | 46/54 |
| 9 | 0.34 | 23 | 31/69 |
| 10 | 0.65 | 5 | 34/66 |
| 11 | 1.33 | 4 | 32/68 |
| 12 | 1.77 | 3 | 29/71 |
| 13 | 2.01 | 17 | 29/71 |

| Example | Ketal Percent | Repellency Oil | Repellency Water |
|---|---|---|---|
| 7 | - | 6 | 6 |
| 8 | - | 6 | 5 |
| 9 | 0.27 | 6 | 6 |
| 10 | 0.16 | 6 | 6 |
| 11 | - | 6 | 6 |
| 12 | 0.09 | 6 | 7 |
| 13 | 0.11 | 6 | 8 |

## EXAMPLES 14 THROUGH 17

The general procedure and approximate reactant and MIBK solvent weights of Example 1 were used to prepare TEA catalyst-free, single charge PFA/PMDA/ECH reaction products at 120 °C. The NMP weight charge was varied; the single charge reaction mixture was heated after admixture to 120 °C in about 25 minutes. The monomer and MIBK/ECH ketal content of and the oil/water repellency imparted to nylon 66 fabric swatches by the corresponding dispersed products are tabulated below.

| Example | NMP Grams | Reaction Time, Hrs. | Monomer/Oligomer Ratio |
|---|---|---|---|
| 14 | 6.67 | 23 | 43/57 |
| 15 | 13.34 | 21 | 39/61 |
| 16 | 19.99 | 21.5 | 37/63 |
| 17 | 26.82 | 6 | 47/53 |

| Example | Ketal Percent | Repellency | |
|---|---|---|---|
| | | Oil | Water |
| 14 | 0.30 | 6 | 5 |
| 15 | 0.22 | 6 | 5 |
| 16 | 0.12 | 6 | 6 |
| 17 | 0.09 | 6 | 5 |

EXAMPLES 18 THROUGH 20

The general procedure and NMP/MIBK weights of Example 1 were used to prepare single charge PFA/PMDA/ECH reaction products at PFA/PMDA mol ratios other than 2.0 and variable reaction times and temperatures.

| Example | Mols | | | | Reaction Temp. °C |
|---|---|---|---|---|---|
| | PFA | PMDA | ECH | TEA | |
| 18 | 0.382 | 0.195 | 0.469 | 0.017 | 120 |
| 19 | 0.412 | 0.216 | 0.541 | 0.018 | 90 |
| 20 | 0.412 | 0.227 | 0.567 | 0.019 | 105 |

| Example | Reaction Time Hours | Monomer/ Oligomer Ratio | Ketal Percent | Repellency | |
|---|---|---|---|---|---|
| | | | | Oil | Water |
| 18 | 20 | 34/66 | 0.06 | 6 | 6 |
| 19 | 22 | 34/66 | 0.10 | 6 | 6 |
| 20 | 18 | 39/61 | 0.14 | 5 | 6 |

EXAMPLE 21

A 500 ml. 4-neck flask (glass) fitted with a paddle stirrer, thermometer, water condenser and an inlet tube for nitrogen and containing a dry (146 ppm water by Karl Fisher analysis) solution of 200 g about 0.405 mole) of PFA in 200 g of NMP was charged with 44.16 g (0.203 mole) of PMDA and 46.81 g (0.506 mole) of ECH. A slow nitrogen purge was started to maintain a dry atmosphere and the stirred mixture heated at a 5° C/10 minute rate to and held at about 120° C for a total reaction period of 20 hours, after which the acid number of the reaction mixture was < 0.44. The reaction product was added to a preheated 85 ° C mixture of 200 g of MIBK, 1000 g of water and 5.87 g of reagent grade ammonium hydroxide and stirred at about 85° C for about 2 hours. After settling, the aqueous supernatant liquid was removed and

replaced by 1000 g of warm water and the wash operation repeated for 50 minutes. After two additional 1000 g water washes, the organic product solution was diluted with 50 g of MIBK and a 400 g portion of the diluted product solution processed as per Example One to obtain the corresponding dispersed product which had a monomer/oligomer ratio of 48/52 and a ketal content of 0.02 percent.

## EXAMPLE 22

The general procedure of Example 1 was followed to dry ( 475 ppm water by Karl Fisher analysis) a wet mixture of 200 g (about 0.403 mole) of PFA in 200 g of NMP, and to then react the resultant PFA solution, 44.06 g (0.202 mole) of PMDA and 46.80 g (0.506 mole) of ECH at 90° C for a total reaction period of 24 hours, after which time the acid number of the mixture was < 0.50. The mixture was cooled to and stirred at 85° C with 3.0 g of reagent grade ammonium hydroxide for one hour before stirring into about 4 L of cold water to precipitate the reaction products as tan solids. After settling, removal of the aqueous supernatant liquid, and two additional water washes, the tacky solids were air-dried and then vacuum oven-dried. A portion of the solids, converted to a stable dispersion by the general procedure of Example 1, had a monomer/oligomer ratio of 58/42 and a ketal content of < 0.005 percent.

## CONTROL

The general procedure and approximate charge weights of Example 1 were used to obtain a relatively dry (246 ppm water by Karl-Fisher analysis) solution of PFA in MIBK/NMP. After cooling the flask contents and modifying the equipment set up as per Example 1, the flask was charged with 44.22 g (0.203 mol) of PMDA and 1.28 g (0.010 mol) of TEA, and the resultant, stirred mixture was heated to and held at reflux for a total reaction period of 23 hours. The reaction mixture was cooled to 50° C and the reaction flask charged with 4.46 g (0.448 mol) of ECH and 0.85 g (0.008 mol) of TEA. The resultant mixture was heated to and stirred at 75° C for a total reaction period of 40.5 hours, after which the acid number of the product mixture was 0.31. The reaction product was stirred with a solution of 5.8 g of reagent grade ammonium hydroxide in 100 g of water at 74-92° C for 0.5 hours. After settling without agitation, the upper aqueous fraction was removed and replaced by 200 g of water and the wash procedure repeated for one hour. After an additional 0.25 hour wash with 100 g of water, the organic product solution was diluted with 100 g of MIBK and 400 g of the solvent-diluted product processed as per Example 1 to obtain an essentially solvent-free dispersion, density 1.2019 by a Metler Digital meter. The oil/water repellency values for nylon 66 fabric treated with the 47/53 monomer/oligomer ratio product after nine home laundry cycles were 5/5.

## Claims

1. A process for the manufacture of bis-(n-perfluoroalkylethyl)-bis-(3-chloro-2-hydroxy-propyl)-pyromellitates which comprises reacting a mixture of pyromellitic dianhydride (PMDA), at least one perfluoroalkylethyl alcohol (PFA) and epichlorohydrin (ECH) in an organic base-containing solvent which is substantially inert to pyromellitic dianhydride, and recovering a mixture of monomeric bis-(n-perfluoroalkylethyl)-bis(3-chloro-2-hydroxypropyl)-pyromellitates and at least one oligomeric product.

2. A process as claimed in claim 1 wherein the mol ratio of PMDA:PFA is in the range between 0.45 and 0.55.

3. A process as claimed in claim 1 or claim 2 wherein the PMDA:ECH mol ratio is in the range between 0.25 and 0.50.

4. A process as claimed in any one of the preceding claims wherein the average reaction temperature is in the range between 70 and 130° C.

5. A process as claimed in any one of the preceding claims wherein a tertiary amine is used to catalyze the reactions.

European Patent Office

# EUROPEAN SEARCH REPORT

Application Number

EP 88 30 4681

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 395 566 (J.H. COVILL)<br>* column 4, lines 10-16; example 1 *<br>--- | 1,2,5 | C 07 C 69/76<br>C 07 C 67/08<br>C 07 C 67/26 |
| A | EP-A-0 095 036 (ALLIED CORP.)<br>* page 8, line 29 page 10, line 18 * &<br>US - A - 4 414 277 (Cat. D)<br>--- | 1,4 | |
| A | EP-A-0 088 389 (AMERICAN HOICHST CORP.)<br>* claims *<br>--- | 1 | |
| A,D | US-A-4 209 610 (F. MARES et al.)<br>* claims *<br>----- | 1 | |
| | | | **TECHNICAL FIELDS SEARCHED (Int. Cl.4)** |
| | | | C 07 C 69/00<br>C 07 C 67/00 |

The present search report has been drawn up for all claims

| Place of search | Date of completion of the search | Examiner |
|---|---|---|
| BERLIN | 19-08-1988 | PROBERT C.L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)